# EUROPEAN PATENT APPLICATION

(11) **EP 2 305 280 A1**
(43) Date of publication of application: **06.04.2011**
(21) Application number: 08764599.0
(22) Date of filing: 23.05.2008
(51) Int. Cl.: A61K 36/48, A61K 8/97, A61Q 19/06

(54) **BCL-2 PROTEIN EXPRESSOR, APOPTOSIS INHIBITOR AND PREVENTIVE FOR UV-DNA DAMAGE IN EPIDERMAL CELLS**

(71) Applicant: Nishihara Co., Ltd., Yamatokoriyama-shi, Nara 639-1015 (JP)
(72) Inventor: TAKITA, Masao, Yamatokoriyama-shi Nara 639-1015 (JP)
(74) Representative: Grünecker, Kinkeldey, Stockmair & Schwanhäusser Anwaltssozietät
(86) International application number: PCT/JP2008/059553
(87) International publication number: WO 2009/141915

(57) **Abstract**

An object of the present invention is to provide an apoptosis inhibiting agent which inhibits apoptosis of a cell having a DNA which is directly or indirectly damaged with ultraviolet-rays or the like, enhances the tissue restoring property of a cell, can be added to various drugs, and can be expected to be widely applied.

An apoptosis inhibiting agent for an epidermal cell, containing a water and ethanol-soluble glycyrrhiza extract as an active ingredient. The apoptosis inhibiting agent has an anti-apoptotic factor due to expression of BLC-2 protein, and has the property of preventing a DNA damage due to ultraviolet-rays. The agent alleviates the DNA damage of an epidermal cell due to ultraviolet exposure and, moreover, does not abnormally or adversely affect a normal skin cell.

## Description

### TECHNICAL FIELD

The present invention relates to a BCL-2 protein expressing agent, an apoptosis inhibiting agent, and an agent for preventing the ultraviolet DNA damage of an epidermal cell, in a cell of the skin or the like.

### BACKGROUND ART

Glycyrrhiza (licorice) which is well known as a crude drug is used for a drug or as a raw material of sweetener, and is also used as an ingredient of skin external preparation such as a cosmetic. In addition, it is known that an extract of a leaf of glycyrrhiza obtained by extraction with aqueous solvents such as water and an aqueous alcohol solution has the action of promoting the production of collagen of skin fibroblasts.

By this action, the glycyrrhiza extract promotes the production of fibrous collagen in dermis to retain the skin tension, and prevents aging of skin by retaining a moisture retaining mechanism by collagen (Patent Document 1).

In addition, it is also known that the glycyrrhiza extract inhibits the production of melanin generated by ultraviolet damage in the skin, and promotes the excretion of melanin, and as the glycyrrhiza extract, a skin cosmetic obtained by extracting the root and rhizome of glycyrrhiza by water, filtering the extract, using the water-insoluble water extraction residue collected as a raw material, and using the ethanol extraction product as an active ingredient is known (Patent Document 2).

Further, as the prior art by the present inventors, a whitening skin external preparation in which the purity of a whitening ingredient contained in glycyrrhiza is increased by purification, particularly whitening ingredients other than liquiritin are carefully selected, that is, the whitening effect is enhanced by the action other than the tyrosinase inhibitory activity is known (Patent Document 3).

Meanwhile, apoptosis regarding skin or other cell death is a life phenomenon which becomes a cause for a variety of diseases, and progresses the symptom of a disease, unlike necrosis, as is referred to as programmed cell death. For this reason, a technique for adjusting apoptosis accompanied with a disease has been demanded, and the development of medicaments has been conducted.

For example, it is well known that when the skin is irradiated with radiation in treatment of a cancerous disease, or when the skin is irradiated with intense sunlight containing ultraviolet-rays routinely, apoptosis is also induced in a normal cell.

Since it is believed that if such apoptosis of a normal cell and the cell of a disease tissue can be inhibited, it serves in the therapeutic effect or in routine strategy for preventing ultraviolet-rays, the development of an apoptosis inhibiting agent has been progressed.

Examples of genes inhibiting apoptosis include BCL-xL, BCL-2, BCL-w and IAP.
Among them, the BCL-2 gene is in chromosome 18q21.3, and is named as "BCL" after initially found follicular lymphoma (B cell lymphoma/leukemia), and an about 26 kD protein encoded by this gene is referred to as "BCL-2 protein".

The BCL-2 protein is expressed in a cytoplasm (including the periphery of nuclear membrane), is present on the outer membrane surface of mitochondria in a normal cell, and acts as an apoptosis inhibiting factor and, particularly, the protein is seen not only in the case of abnormality such as a tumor cell, but also in the nerve system, intestine mucosa, and an epidermal base layer in a normal cell.

The BCL-2 gene is a gene essential for maintaining a pigment stem cell, and it is known that whitening of hair occurs due to its defect. That is, the pigment stem cell of follicle is grown together with a matured pigment cell producing melanin, however, according to an experimental result using a BCL-2-deficient mouse, the BCL-2 gene is essential for existence at an instant when melanoblast localized in a niche via development enters the dormant state as a pigment stem cell.
It is known that, in the BCL-2 gene-deficient mouse, the enhancement of apoptosis in a variety of tissues such as nerve system, lymphocyte and small intestine and a symptom such as renal failure, including whitening of body hair due to apoptosis of melanocyte are seen, and it is know that the BCL-2 function is important for maintaining these tissues and cells (Patent Document 4).
BCL-2 is normally expressed in many tissues of the body, and plays a physiological role for maintaining the existence of a long life cell therein. It is known that, as a type of a cell, existence of which is regulated by BCL-2, there are "memory" formed during immunization, and a stem cell which becomes an absorptive cell present in lymphocyte, brain, muscle and many types of neurons in peripheral nerve controlling the function of organs, bone marrow, skin, and the inside of stomach intestine tract (Patent Document 5).

Patent Document 1: JP-ANo.2000-191498 gazette (claim 1)
Patent Document 2 JP-A No. 10-194959 (paragraph 0005, paragraph 0014)
Patent Document 3: JP-A No. 2006-028157 gazette (paragraph 0016)
Patent Document 4: JP-A No. 2002-080382 gazette
Patent Document 5: JP-A No. 2003-176236 gazette

### DICLOSURE OF THE INVENTION

### PROBLEMS TO BE SOLVED BY THE INVENTION

However, regarding of the property of expressing the BCL-2 protein or the property of inhibiting apoptosis, there is no finding associated with the aforementioned glycyrrhiza extract, the whitening action and aging preventing action known in Patent Documents 1 to 3 are due to the action of promoting collagen production and inhibiting melanin production on a live cell unlike the action exerted by anti-apoptosis due to the expression of BCL-2 protein (hereinafter, abbreviated as expression of BCL-2, or BCL-2 expression in some cases).

As described above, the technique of utilizing an aqueous solvent extract of glycyrrhiza has a problem that the property of expressing the BCL-2 protein and the inhibition of abnormal apoptosis of an epidermal cell damaged with ultraviolet-rays or the like has not been technically established.

Then, an object of the present invention is to provide a BCL-2 protein expressing agent, an apoptosis inhibiting agent, and an agent for preventing the ultraviolet DNA damage of an epidermal cell, which solves the aforementioned problems, enhances the expression of the BCL-2 protein of a cell, further, inhibits apoptosis of a cell having a DNA directly or indirectly damaged with ultraviolet-rays or the like, enhances the tissue restoring property of a cell, can be added to a variety of drugs, and can be expected to be widely applied.

### SOLUTION TO THE PROBLEMS

The BCL-2 protein expressing agent and apoptosis inhibiting agent of the present invention have been completed by the present inventors through finding based on an experimental method described later and the results thereof, of nature such that proliferation of an epidermal cell (PCNA), the expression of BCL-2, and the property of inhibiting apoptosis of an epidermal cell are recognized, respectively, and the agent alleviates the DNA damage of an epidermal cell due to ultraviolet exposure and, moreover, does not abnormally or adversely affect a normal skin cell.

That is, in the present invention, the aforementioned problems were solved by adopting a BCL-2 protein expressing agent, an apoptosis inhibiting agent, or an agent for preventing the ultraviolet DNA damage of an epidermal cell, containing a water and ethanol-soluble glycyrrhiza extract as an active ingredient.

The glycyrrhiza extract adopted as an active ingredient in the present invention is harmless and safe to a normal epidermal cell, that is, does not significantly affect the cell proliferating ability and apoptosis in a normal epidermal cell and, even when added to a cosmetic or the like, does not remarkably affect the action of inhibiting apoptosis to a normal epidermal cell.
That is, by the previous method of using a cosmetic, glycyrrhiza was not actively used as a BCL-2 protein expressing agent, an apoptosis inhibiting agent, or an agent for preventing the ultraviolet DNA damage of an epidermal cell, and the action thereof was not assuredly exerted.

Meanwhile, for a cell in which a damage was caused in a DNA by ultraviolet irradiation, the BCL-2 protein expressing agent, the apoptosis inhibiting agent or the agent for preventing an ultraviolet DNA damage significantly proliferates an epidermal cell to restore a tissue, expresses BCL-2 which is an apoptosis inhibiting factor, and inhibits formation of a thymine dimer which damages a DNA.
The property of preventing the DNA damage of an epidermal cell is also clear by the fact that the appearance of a T-T dimer positive cell is significantly more reduced in a treatment group than in a non-treatment group, as shown in Table 3 described later.

Such a BCL-2 protein expressing agent, an apoptosis inhibiting agent or an agent for preventing a DNA damage is effective for an epidermal cell, prevents the DNA damage of an epidermal cell, expresses the BCL-2 protein, and inhibits apoptosis.

As described above, it is believed that, according to the BCL-2 protein expressing agent for the skin to a pigment stem cell, it acts such that the BCL-2 gene is supplied at a term necessary for existence of a pigment stem cell and, as a result, melanin is produced by maintaining a pigment stem cell in an epidermal cell, and physiological whitening of hair accompanied with aging can be prevented.
In addition, the BCL-2 protein expressing agent of the present invention can be used by various formulations in which improvement is recognized by the expression of the protein through the BCL-2 gene in various tissues and cells of human and animal, and the agent has general-purpose properties such that it can be applied to, for example, organ therapy, improvement in various symptoms due to a longer life of a variety of cells including a stem cell, health maintenance, cosmetic improvement in the skin and hair and other various formulations.

### ADVANTAGES OF THE INVENTION

Since the present invention is a BCL-2 protein expressing agent or an apoptosis inhibiting agent containing a water and ethanol-soluble glycyrrhiza extract as an active ingredient, there is an advantage that the agent becomes a BCL-2 protein expressing agent, an apoptosis inhibiting agent or an agent for preventing the ultraviolet DNA damage of an epidermal cell, which inhibits apoptosis of a cell having a DNA which is directly or indirectly damaged with ultraviolet-rays or the like, enhances the tissue restoring property of a cell, and is added to various drugs, and can be expected to be widely applied.
In addition, the BCL-2 protein expressing agent of the present invention also has an advantage that it also has the effect of prolonging a life of a somatic cell other than a skin cell, and has the effect of improving health depending on localization of such a cell.

### BEST MODE FOR CARRYING OUT THE INVENTION

The glycyrrhiza used in the present invention is a crude drug obtained by drying glycyrrhiza which is a papilionaceous medical plant produced in Spain, Siberia and China to enhance the storage property, and the plant is a plant or its related species having a scientific name of Glycyrrhiza urarelensis Fischer or Glycyrrhiza glabra 1 var. glandulifera REG. Et HERD. A material obtained by milling or grinding, preferably, a root or a rhizome (also referred to as creeping stem or stolon) of the plant with the skin or with the cork skin removed, into a powder, or a material obtained by extracting them with water or ethanol or other aqueous solvents can be used as an extraction material of an active ingredient. Safety of a general glycyrrhiza extract to the skin is well known.

A solvent used for extracting the active ingredient in the present invention is water (which may be any of neutral, mildly acidic or mildly alkaline), or an aqueous extraction solvent in which water is used as a main component, and other solvents (which may be organic solvents) are appropriately added thereto, which is an aqueous solution containing alcohol may be adopted. Examples of the alcohol include well-known alcohol solvents such as methanol, ethanol, isopropanol and t-butanol. In addition, active ingredients extracted with these solvents are all soluble in water and ethanol (ethyl alcohol).

It is preferable that extraction operation with such an aqueous solvent is performed in the state of warm water or hot water in order to enhance extraction efficiency. When extraction operation is performed at particularly 100°C or higher, preferably 100 to 130°C, ingredients effective for BCL-2 protein expression, apoptosis inhibition or prevention of the ultraviolet DNA damage of an epidermal cell are efficiently extracted.

It is preferable that, in an extraction step, an extract solution is filtered with an adsorbent such as active carbon, or is subjected to solid liquid separation utilizing a difference in specific gravity between a solid and a liquid or a difference in settling rate due to a gravity force or a centrifugal force, to remove suspending substances.

As the adsorbent, a substance which provides an interface that causes much positive adsorption, such as active carbon may be used, and it is preferable that the thus obtained transparent liquid is concentrated, this is diluted again with water or ethanol, insolubles generated by concentration are separated and removed by filtration, thereby, purification is made to be more complete. Examples of the adsorbent include synthetic adsorbents (organic adsorbents) in addition to inorganic adsorbents comprised of a porous material such as active carbon.

Examples of the inorganic adsorbents include metal oxides, metal reduced products, and metal hydroxides such as active alumina, silica gel, and titanium oxide, clay minerals such as bentonite and acid clay, and hydrous silicate minerals having the cleavage property such as diatomaceous earth and talc.

Active carbon is not such that it is used by particularly strictly selecting the material quality thereof, and active carbon using charcoal, bamboo and palm shell as raw materials has good efficiency of adsorbing suspending substances comprised of fine particles, and is preferable.

The synthetic adsorbent is comprised of particles made of an ion-exchange resin in which fine continuous pores are formed up to the interior of the particle, and includes an aromatic crosslinked styrene-based porous polymer, a substituted aromatic adsorbent in which a bromine atom is bonded to the aromatic nucleus of an aromatic polymer, and an acrylic hydrophilic adsorbent having a methacrylic acid ester polymer as a skeleton.

As the solid liquid separation and other operations for removing suspending substances as described above, a method of passing an extraction solution through a container such as a column filled with active carbon particles, or adding an active carbon powder (and a filtration aid such as talc, if necessary) to an extraction solution to be stirred, and filtering the mixture with a filter capturing the active carbon powder particles such as filter paper or a filter cloth can be also adopted and, in addition, centrifugation, and the well-known liquid separation means due to a difference in settling rate may be adopted.

As the concentration operation on an aqueous solvent extract, a well known concentration means such as a pressure reducing operation or heat concentration can be adopted. A concentration ratio is preferably around 2 to 100-fold, particularly around 5 to 50-fold, usually, around 10-fold concentration is preferable.

It is preferable that the resulting concentrate is diluted again with the aforementioned aqueous solvent extraction liquid or an aqueous solvent equivalent thereto. It is not necessary that a dilution ratio is particularly limited, but it is believed that a dilution ratio to such an extent that the volume is returned to that before concentration is preferable, and the dilution ratio is around 2 to 100-fold. It is preferable that two or more steps are repeated, setting the operation of concentration and dilution to be one step.

In order to further purify the thus obtained liquid, it is preferable to use absolute alcohol having an ethanol concentration of 99.5% or more as an extraction solvent after concentration.

In order to prepare an ethyl alcohol-soluble ingredient obtained by solid liquid separation as a skin external preparation, if the ingredient is mixed with a base for a skin external preparation having a good skin adhering property, which has suitable hydrophilicity / lipophilicity, or an extract solution is mixed with a base such as cream, ointment, and lotion, the ingredient can be prepared into a preparation form which can be applied on the skin.

The preparation forms of the BCL-2 protein expressing agent, the apoptosis inhibiting agent and the agent for preventing the ultraviolet DNA damage of an epidermal cell of the present invention are not particularly limited, but for example, a form well known as an external preparation can be adopted, and it is preferable to formulate into a preparation form which is easily applied on the skin, such as a water soluble liquid, lotion, cream, ointment, and powder.
In addition to use as the cosmetic, the BCL-2 protein expressing agent of the present invention can be also formulated into preparation forms of a medicament and a quasi drug such as injectables, inhalations, oral preparations, suppositories and ointments, and can be also used by mixing a well known ingredient which is usually used in each preparation.

As described later, it is preferable that an active ingredient as the BCL-2 protein expressing agent, the apoptosis inhibiting agent and the agent for preventing the ultraviolet DNA damage of an epidermal cell of a water and ethanol-soluble glycyrrhiza extract is blended so that the final concentrate is formulated at a concentration of 0.1 to 10% by weight to the skin, as expressed by a blending weight % as a dried substance (dry solid matter).

### EXAMPLES

### [Process for actually producing glycyrrhiza extract solution (T)]

1) Water (2000 liters (hereinafter, liter is represented by L)) was added to 200 kg of cut Glycyrrhiza urarelensis Fischer, this was extracted at 95°C for 2.5 hours and the extraction solution was filtered with a vibration suppressing sieve (300 mesh, made of stainless). The resulting extraction filtrate was concentrated (liquid temperature 60°C or lower, under reduced pressure), and the concentrate (spray dry stock solution) was spray-dried to obtain a dry extract. This was sieved (60 mesh, made of stainless) to obtain a yield of 20 kg of a glycyrrhiza dry extract (T origin).
2) Pharmacopoeia grade ethanol (100 L) was added to 20 kg of the Glycyrrhiza urarelensis Fischer dry extract (T origin) obtained in 1), and the mixture was stirred for 6 hours, and filtered with No. 2 filter paper.
3) Pharmacopoeia grade ethanol was added to the filtrate to be 100 L, 4.5 kg of active carbon (particulate Shirasagi KL) was added thereto, and the mixture was stirred for 3 hours, and filtered with No. 2 filter paper. This step was repeated 3 times.
4) Pharmacopoeia grade ethanol was added to the filtrate obtained in 3) to be 100 L, and this was concentrated (50°C or lower, under reduced pressure) to be 10 L. Further, 400 g of active carbon (particulate Shirasagi KL) was added thereto, and the mixture was stirred for 2 hours, and filtered with No. 2 filter paper. This step was repeated 6 times.
5) Pharmacopoeia grade ethanol was added to the filtrate obtain in 4) to be 10 L, and this was concentrated to be 2 L. To this were added 1 L of pharmacopoeia grade ethanol and 100 g of active carbon (particulate Shirasagi KL), and the mixture was stirred for 3 hours and filtered with No. 2 filter paper.
6) Pharmacopoeia grade ethanol was added to the filtrate obtained in 5) to be 2L, 100 g of active carbon (particulate Shirasagi KL) was added thereto, and the mixture was stirred for 3 hours and filtered with No. 2 filter paper.
7) Pharmacopoeia grade ethanol was added to the filtrate obtained in 6) to be 2L, followed by filtration with No. 5 filter paper.
8) The filtrate obtained in 7) was concentrated (40°C or lower, under reduced pressure) to dryness, 300 mL of water was added thereto, this was extracted (shaking, at room temperature) and filtered with No.5 filter paper. This step was performed 3 times.
9) The filtrate obtained in 8) was adjusted to 1 L.

The filtrate (250mL) obtained in 9) was heated to 95 to 110°C by boiling in hot water to obtain a concentrate (37.1 g), to this was added 1 L of 99.5% absolute ethanol (Konishi Co., Ltd.), ethanol-soluble fractions were collected, which was heated to 95 to 100°C to obtain a concentrate (21.5 g). To this were added 50 mL of purified water and 50 g of active carbon (manufactured by Wako Pure Chemical Industries, Ltd. :active carbon powder), the mixture was stirred and, at the same time, 75 g of talc (manufactured by Maruishi pharmaceutical Co., Ltd.) was added, and the mixture was further stirred and filtered with filter paper. The resulting filtrate was heated to 95 to 100°C to obtain a concentrate (14.7 g), to this was added 400 ml of 99.5% absolute ethanol (Konishi Co., Ltd.), ethanol-soluble fractions were collected, this was heated to 95 to 100°C to obtain a concentrate (11.7 g), to this were added 50 mL of purified water and 30 g of active carbon (manufactured by Wako Pure Chemical Industries, Ltd.: active carbon powder), the mixture was stirred and, at the same time, 50 g of talc (manufactured by Maruishi pharmaceutical Co., Ltd.) was added, followed by further stirring. Thereafter, the mixture was filtered with filter paper, the resulting filtrate was heated to 95 to 100°C to obtain a concentrate (8.6 g), to this was added 400 ml of 99.5% absolute ethanol (Konishi Co., Ltd.), ethanol-soluble fractions were collected, and this was heated to 95 to 100°C to obtain a concentrate (5.2 g). To 2.0 g of this concentrate was added purified water to be 60 g, which was used as a sample.

Regarding the active ingredient of the glycyrrhiza extract, it is believed to be effective for obtaining the effect of the present invention that the final concentrate (5.2g) is contacted with the skin in a range of the concentration of 0.1 to 10% by weight, in terms of blending weight% of a dried substance.

### [Method of assessment experiment (1)]

1) Four-week old C56BL6 male mice (Japan SLC, Inc.) were habituated for 1 week, hair was shaved, and grouping was performed. An experiment group is five groups from First group to Fifth group, and 15 mice per group were used.
   First group (Comparative Example): PBS application before and after ultraviolet irradiation
   Second group (Example): Sample application before ultraviolet irradiation
   Third group (Example): Sample application after ultraviolet irradiation
   Fourth group (Example): Sample application before and after ultraviolet irradiation
   Fifth group (Comparative Example): Only shaving
2) The sample was stored at 4°C, and returned to room temperature before application.
3) In ultraviolet irradiation, full wavelength ultraviolet-rays (UV-A, -B, -C) were irradiated to induce an acute ultraviolet damage on the skin, thereupon, the sample was applied on the mouse skin 24 hours before ultraviolet irradiation or immediately after irradiation, and change in the acute ultraviolet damage was studied.
4) A shaving site is 2 cm square centering an intersection between a costate rib thoracic vertebra inferior margin and spondylus, and a shaving area is 4 cm². Shaving was performed using an electric shaver (National), and a razor was not used.
5) An ultraviolet-ray was irradiated for 1.5 minutes at an ultraviolet-ray intensity of 11 mW/cm² (irradiation distance 5 cm) using an ultraviolet generating apparatus (XX-15BLB, UVP Co, Tokyo, Japan). An ultraviolet irradiation dose thereupon was 1.0 J/cm².
6) Upon irradiation, a mouse was anesthetized with nembutal (mg/mouse intravenous injection) and, thereafter, extremities were fixed with a tape to retain resting of posture during irradiation.
7) The sample (200 µl) was applied on a shaved part with a cotton swab at an all amount, while drying.
8) A mouse was sacrificed by cervical vertebra dislocation 24 hours after ultraviolet irradiation, the skin of a shaved part including its surrounding non-shaved part was peeled on a fascia, spread on a rubber plate, and fixed with 10% formalin for 24 hours.
9) As a skin section, a tissue having a width of 4 mm was cut out from a central part of a specimen, and a paraffin-embedded block was prepared to prepare a thin section specimen having a thickness of 3 µm.
10) Hematoxylin nuclear staining (hereinafter, also abbreviated as HE staining) and immunological staining were performed from the thin section specimen.
11) For immunological staining, the following antibodies were used, and antigen activation and dilution were performed as in tables. Optimax Wash Buffer (Biogenex) was used for antigen dilution and washing.

| Antigen name | Clone | Treatment | Concentration |
|---|---|---|---|
| PCNA(DAKO) | PC10 | Citric acid-MW | 1/50 |
| BCL 2 (DAKO) | | Pepsin | 1/100 |
| ss-DNA (IBL) | | Pepsin | 1/200 |
| T-T Dimer (donation) | | Pepsin | 1/200 |

12) A protocol for immunological staining is as follows.
1: Deparaffinization, water addition
2: Antigen activation
3: 0.3%H₂O₂-methanol treatment
4: Primary antibody treatment
5: Washing
6: Secondary antibody treatment
7: Washing
8: DAB color development
9: Hematoxylin nuclear staining
10: Sealing

13) For assessing tissue pathology by HE staining, the confirmation of a toxicologic pathology certifying physician was obtained.
14) Determination of immunological staining
   (1) Regarding PCNA, ssDNA and a D-D dimer, 500 cells including all layers of an epidermal cell were observed under a microscope, and a cell having a positive finding in a nucleus was defined as positive.
      Incidentally, PCNA (proliferating cell nuclear antigen) exhibits the cell proliferating ability, is relatively stable throughout a cell cycle, is stable at a very low level in a non-proliferating cell, but is rapidly increased when enters a cell division cycle and, therefore, it becomes a label which can detect transfer from a non-proliferation cell population to a proliferating cell population.
   (2) Regarding BCL-2, when compared with BCL-2 expression at a non-shaved part skin (=non-ultraviolet irradiation part) in a specimen, one exhibiting a stronger immunological reaction was defined as positive. A case where a staining intensity is lower than that of a non-irradiation part epidermis was defined as negative, the same degree to 2-fold or less was defined as positive, and 2-fold or more was defined as strongly positive.

### [Experimental result]

Regarding morphological change by the aforementioned experimental methods, the results are shown in Table 1. That is, the influence of the sample on change in an epidermal cell in mouse skin ultraviolet irradiation was morphologically examined.

**[Table 1]**

| Group | Treatment | Epidermal cell layer | Inflammatory cell infiltration |
|---|---|---|---|
| First group | Before and after UV (PBS) | 3.6 ± 1.8 | ++ |
| Second group | Before UV (sample) | 4.2 ± 2.0 | + |
| Third group | After UV (sample) | 7.8 ± 3.3 | + |
| Fourth group | Before and after UV (sample) | 8.6 ± 3.5 | + |
| Fifth group | Only shaving | 3.1 ± 1.2 | - |

| | | | |
|---|---|---|---|
| *PBS = Phosphate buffer saline | | | |

As also apparent from the results shown in Table 1, a normal mouse epidermis was comprised of one layer of a base cell layer and one to two layers of stratum spinosum. In Fifth group (only shaving), this normal finding was retained, and no inflammatory cell was recognized.
In First group (irradiation + PBS treatment), the fundamental construction of epidermis was retained, and apparent erosion formation and epidermal cell necrosis were not recognized. However, mild inflammatory cell infiltration was seen from the superficial layer of epidermis to within epidermis, and this was regarded as inflammatory change accompanied with ultraviolet irradiation.
In Second group (irradiation + sample application before irradiation), morphological change in epidermis was not seen, and inflammatory cell infiltration was milder than First group.
In Third group (irradiation + sample application after irradiation) and Fourth group (irradiation + sample application before and after irradiation), epidermis was thickened into 8 to 10 layers due to mainly increase in stratum spinosum, but atypia was not recognized in constituent cells, and tissue construction was also retained. Inflammatory cell infiltration was milder than First group (irradiation + PBS treatment).
In addition, regarding inflammatory cell infiltration, since it was seen in First group (irradiation + PBS treatment), inflammatory cell infiltration was believed to be an inflammation reaction by a cell damage due to ultraviolet irradiation. On the other hand, inflammatory cell infiltration was decreased in sample application groups, particularly, in Third group and Fourth group in which application after irradiation was performed, and it was recognized that inflammation was inhibited by sample application.

Regarding the proliferating ability of an epidermal cell, the influence of the sample on change in an epidermal cell in mouse skin ultraviolet irradiation was investigated with respect to the cell proliferating ability and apoptosis.
That is, the proliferating ability of an epidermal cell was studied by the PCNA positive cell number through immunological staining.
Regarding apoptosis of an epidermal cell, a frequency of positive cells in which a DNA was fragmented (apoptosis cell (%)) was determined by a single strand DNA (ssDNA) immunological staining method, and the results thereof were also described in Table 2.

**[Table 2]**

| Group | Treatment | PCNA-labeled cell (%) | Apoptosis cell (%) |
|---|---|---|---|
| First group | Before and after UV (PBS) | 14 ± 5 | 32 ± 12 |
| Second group | Before UV(sample) | 13 ± 4 | 24 ± 8 |
| Third group | After UV (sample) | 15 ± 6 | 6 ± 6 |
| Fourth group | Before and after UV(sample) | 17 ± 6 | 4 ± 4 |
| Fifth group | Only shaving | 6 ± 4 | 3 ± 2 |

| | | | |
|---|---|---|---|
| *PBS = Phosphate buffer saline | | | |

As apparent also from the results of Table 2, in Fifth group (only shaving) of the PCNA positive cell, the PCNA-labeled cell was seen only in a base cell layer, and the positive frequency was 6±4%. To the contrary, First group (irradiation + PBS treatment), Second group (irradiation + sample application before irradiation), Third group (irradiation + sample application after irradiation) and Fourth group (irradiation + sample application before and after irradiation), in which ultraviolet irradiation was performed, the PCNA positive frequency was 14±5%, 13±4%, 15±6%, and 17±6%, respectively, and a significant difference was recognized between Fifth group (only shaving) and other Groups (P<0.001). However, in Second to Fourth groups in which the sample was applied, a similar increase of the PCNA positive cell to that of First group in which application was not performed was recognized, and it was believed that therefrom, when ultraviolet irradiation was performed, a cell tissue restoring reaction against a cell damage occurs in any case.

Regarding apoptosis of an epidermal cell, in Fifth group (only shaving), the small number of ssDNA positive cells were seen in stratum spinosum, being 3±2%. To the contrary, in First group (irradiation + PBS treatment) in which ultraviolet irradiation was performed, the ssDNA positive cell was 32±12%, being highly frequency.

On the other hand, in Second group (irradiation + sample application before irradiation) in which the sample was applied before irradiation, the ssDNA positive apoptosis cell frequency was 24±8%, being significantly higher frequency than that of Fifth group as a non-irradiation control.
In addition, in Third group (irradiation + sample application after irradiation) and Fourth group (irradiation + sample application before and after irradiation) in which the sample was applied after irradiation, the ssDNA positive apoptosis cell frequency was 6±6% and 4±4%, respectively, being significantly reduced more than First group (irradiation + PBS treatment) and Second group (irradiation + sample application before irradiation), and a significant difference was not seen with respect to Fifth group (only shaving).

When these results are generalized, in Third and Fourth groups in which the sample of Example was applied after irradiation, apoptosis of an epidermal cell was inhibited, and it was believed that both of reactive proliferation property changes acted synergistically, thereby, thickening of epidermis was seen.

Then, regarding the expression of BCL-2 in an epidermal cell, the influence of the sample on change in an epidermal cell in mouse skin ultraviolet irradiation was investigated with respect to BCL-2 expression and ultraviolet DNA damage, and the expression of BCL-2 which was widely recognized as a factor inhibiting apoptosis was investigated by immunological staining, and the results were shown in Table 3.

**[Table 3]**

| Group | Treatment | BCL-2 expression | T-T dimer positive cell (%) |
|---|---|---|---|
| First group | Before and after UV (PBS) | Faint | 16 ± 8 |
| Second group | Before UV (sample) | ++ | 9 ± 6 |
| Third group | After UV (sample) | +++ | 4 ± 4 |
| Fourth group | Before and after UV (sample) | +++ | 4 ± 3 |
| Fifth group | Only shaving | Faint | 0 |

| | | | |
|---|---|---|---|
| *PBS = Phosphate buffer saline | | | |

BCL-2 expression was seen very faintly in a base layer in Fifth group (only shaving). Faint expression was seen in a base layer also in First group (irradiation + PBS treatment) in which ultraviolet irradiation was performed, but a clear difference with respect to Fifth group was not recognized.
On the other hand, in Second group (irradiation + sample application before irradiation) in which the sample was applied before irradiation, BCL-2 expression was positive, and both of an expression intensity and the positive cell number were significantly enhanced than Fifth group as a control and First group of only irradiation. To the contrary, in Third group (irradiation + sample application after irradiation) and Fourth group (irradiation + sample application before and after irradiation) in which the sample was applied after irradiation, BCL-2 expression was strongly positive in both cases, and a clear difference was recognized between First group (irradiation + PBS treatment), Second group (irradiation + sample application before irradiation) and Fifth group (only shaving).

Regarding a DNA damage due to ultraviolet irradiation in an epidermal cell, since it is known that formation of a thymine dimer (T-T dimer) is generated in an intranuclear DNA by an ultraviolet-ray, particularly, high energy UVC, formation of a T-T dimer after irradiation was studied using an anti-T-T dimer antibody.

The T-T dimer positive cell was not recognized at all in Fifth group (only shaving). To the contrary, in First group (irradiation + PBS treatment) in which ultraviolet irradiation was performed, the positive cell was highly frequent, such as 16±8%.
On the other hand, in Second group (irradiation + sample application before irradiation) in which the sample was applied before irradiation, the T-T dimer cell frequency was 9±6%, which was a significantly higher frequency than Fifth group as a non-irradiation control.
To the contrary, in Third group (irradiation + sample application after irradiation) and Fourth group (irradiation + sample application before and after irradiation) in which the sample was applied after irradiation, the T-T dimer cell frequency was 4±4% and 4±3%, respectively, being significantly reduced more than First group (irradiation + PBS treatment) and Second group (irradiation + sample application before irradiation), and a significant difference was not seen with respect to Fifth group (only shaving).

From the foregoing, it is recognized that BCL2 is significantly expressed by application of sample, and a DNA damage due to ultraviolet irradiation is inhibited by sample application (particularly, sample application after ultraviolet irradiation). In addition, since BCL-2 has no efficacy of inhibiting a DNA damage, it is thought that sample application exerts BCL-2 expression and the effect of inhibiting a DNA damage, separately.
And, a DNA damage due to formation of a T-T dimer was evoked in epidermis by ultraviolet irradiation, and it was confirmed that, for this reason, apoptosis is induced, and the proliferating ability is enhanced as a restoration reaction.

On the other hand, in Third group Fourth group in which the sample was applied after irradiation, inhibition of apoptosis was seen and, as a cause therefor, enhancement of BCL 2 expression having anti-apoptotic action, and decrease in a T-T dimer are thought. To the contrary, the epidermis proliferating ability is enhanced as in a non-applied group, and it was thought that morphological change in hyperplasia property of epidermis, that is, thickening is shown.

In Second group in which the sample was applied before irradiation, a value was between that of Third group of only application after irradiation and that of Fifth group of non-application irradiation. In addition, in Fourth group in which application was performed before and after irradiation, the additive effect was not recognized as compared with Third group. As a cause therefor, incompleteness of administration due e.g. to the subjects removing the sample by licking after application was thought as a possibility, but it was also thought that action remains mild on an epidermis at non-stimulation.

### (Assessment experiment 2): Effect of sample on normal skin

In Experiment 1, the effect of sample in the skin in the stress state of ultraviolet irradiation was studied, but in order to see the action of sample at non-stress, the sample was applied on the skin which had been subjected to only shaving. Change in a relatively long term continuous administration protocol of performing sample application three times/week for 4 weeks was assessed by morphological study and with immunological staining in Experiment 1.

### [Experimental method]

1) Four-week old C56BL6 male mice (Japan SLC Inc.) were habituated for 1 week, and grouping was performed after shaving. An experimental group was two groups of sample application group and PBS application group, and 15 mice per group were used.
   First group (Comparative Example): PBS application
   Second group (Example): Sample application
2) A sample was stored at 4°C, and was returned to room temperature before application.
3) A shaving site is 2 cm square centering in an intersection between a costate rib thoracic vertebra inferior margin and spondylus, and a shaving area is 4 cm². Shaving was performed using an electric shaver (National), and a razor was not used.
4) A sample (200 µl) was applied on a shaved part with a cotton swap at an all amount, while drying. Coating was performed once a day, at 9 o'clock at which activity is not high, three times/week for 4 weeks.
5) A mouse was slaughtered by cervical vertebra dislocation 24 hours after ultraviolet irradiation, the skin of a shaved part including its surrounding non-shaved part was peeled on a fascia, spread on a rubber plate, and fixed with 10% formalin for 24 hours.
6) As a skin section, a tissue having a width of 4 mm was cut out from a central part of a specimen, and a paraffin-embedded block was prepared to prepare a this section specimen having a thickness of 3 µm.
7) HE staining and immunological staining were performed form a thin section specimen.
8) In immunological staining, the same study as that of Experiment 1 was performed.
9) A protocol of immunological staining was the same as that of Experiment 1.
10) For assessing tissue pathology by HE staining, confirmation of a toxicologic pathology certifying physician was obtained.
11) Determination of immunological staining. This was performed as in Experiment 1.

### [Experimental result]

1) Regarding morphological change, the influence of sample on change in an epidermal cell in a normal skin of a mouse was morphologically investigated, and results thereof are shown in Table 4.

**[Table 4]**

| Group | Treatment | Epidermal cell layer | inflammatory cell infiltration |
|---|---|---|---|
| First group | PBS application | 13 ± 4 | - |
| Second group | Sample application | 13 ± 4 | - |

### 1) Morphological change

When a sample application group and a PBS application group as a control were compared, there was no difference in morphology of epidermis, and normal construction was retained. In a applied group, change in hyperplasia and hyperkeratosis of epidermis was not seen, and inflammation change such as inflammatory cell infiltration was also not recognized.

### 2) Epidermal cell proliferating ability

The PCNA positive cell frequency in First group (PBS application) and Second group (sample application) was 6±4% and 5±4%, respectively, and a significant difference was not recognized. These values are the same as those of Fifth group (only shaving) of Experiment 1, and it was thought that sample application does not give significant change to the cell proliferating ability, in the ultraviolet non-irradiation state.

3) Regarding apoptosis of an epidermal cell, the influence of sample on cell proliferation and apoptosis of a normal skin was investigated, and the results thereof are shown in Table 5.

**[Table 5]**

| Group | Treatment | PCNA-labeled cell (%) | Apoptosis cell (%) |
|---|---|---|---|
| First group | PBS application | 6 ± 4 | 8 ± 5 |
| Second group | Sample application | 5 ± 4 | 4 ± 4 |

As also apparent from results of Table 5, the ssDNA positive apoptosis cell frequency in First group (PBS application) and Second group (sample application) was 8 ± 5% and 4 ± 4%, respectively, and a difference was not recognized between both of them. In addition, the value was a value equivalent to that of Fifth group (only shaving), and it was thought that sample application does not give significant change to apoptosis in the ultraviolet non-irradiation state.

Regarding expression of BCL-2 in an epidermal cell, the effect of sample on a normal skin was investigated with respect to BCL-2 expression and an ultraviolet DNA damage (formation of a T-T dimmer), and the results thereof are shown in Table 6.

**[Table 6]**

| Group | Treatment | BCL-2 expression | T-T dimer positive cell (%) |
|---|---|---|---|
| First group | PBS application | Faint | 0 |
| Second group | Sample application | + | 0 |

As also apparent from the results of Table 6, mild enhancement of BLC-2 expression was recognized in a sample application group as compared with a PBS application group as a control, but enhancement of BLC-2 expression was extremely mild as compared with a group of application after ultraviolet irradiation of Experiment 1, and this corresponded to no remarkable change in an apoptosis frequency.
Regarding formation of a T-T dimer in an epidermal cell, formation of a T-T dimer was not recognized on epidermis, in any group, under condition of no ultraviolet irradiation.

As also apparent from the above results, it was recognized that samples of Examples make an epidermal cell of a living body express the BCL-2 protein, and inhibit apoptosis. In addition, in Examples, an amount of formation of a T-T dimer in an epidermal cell to which an ultraviolet-ray had been irradiated was decreased, and it was recognized that a DNA damage due to ultraviolet irradiation is prevented.

## Claims

1. A BCL-2 protein expressing agent, comprising a water and ethanol-soluble glycyrrhiza extract as an active ingredient.

2. A BCL-2 protein expressing agent for the skin, wherein the expression of BCL-2 protein is the expression of BCL-2 protein in an epidermal cell, in the BCL-2 protein expressing agent as defined in claim 1.

3. The BCL-2 protein expressing agent for the skin according to claim 2, wherein the epidermal cell is a pigment stem cell.

4. An apoptosis inhibiting agent, comprising the BLC-2 protein expressing agent as defined in claim 1 or 2 as an active ingredient.

5. An agent for preventing the ultraviolet DNA damage of an epidermal cell, comprising the glycyrrhiza extract as defined in claim 1 as an active ingredient.
